# EUROPEAN PATENT APPLICATION

(11) **EP 2 584 317 A1**
(43) Date of publication of application: **24.04.2013**
(21) Application number: 11795226.7
(22) Date of filing: 03.06.2011
(51) Int. Cl.: G01C 22/00, A43B 5/06, A61B 5/103

(54) **INTELLIGENT PROGRAMMABLE SHOE**

(30) Priority: 16.06.2010 ES 201000830
(71) Applicant: Calzados Hergar, S.A., 26580 Arnedo (La Rioja) (ES)
(72) Inventor: GARCIA MORON, Basilio, E-26580 Arnedo La Rioja (ES); GARCIA MORON, Ivan, E-26580 Arnedo La Rioja (ES); GARCIA MORON, Julio, E-26580 Arnedo La Rioja (ES); HERNANDEZ STARK, Rafael, E-26580 Arnedo La Rioja (ES); MONTIEL PARREÑO, Enrique, E-26580 Arnedo La Rioja (ES); NAVARRO SALA, Rocio, E-26580 Arnedo La Rioja (ES); POVEDA VERDU, Damian, E-26580 Arnedo La Rioja (ES); RIZO VICEDO, Wigberto, E-26580 Arnedo La Rioja (ES)
(74) Representative: Villamor Muguerza, Jon
(86) International application number: PCT/ES2011/000201
(87) International publication number: WO 2011/157870

(57) **Abstract**

The invention relates to a programmable intelligent shoe especially designed for its user to have a control of different variables which are presented when walking, such as the distance traveled, the time used, the calories consumed, etc, the shoe has a sole (2), suitable with any line of design, a sole in which a practicable package (4) is provided, internally provided with an electronic circuit (5), in which a step detector (6) is provided according to the time and an emitter (7), such that the mentioned shoe is complemented with a receiver device (8) wirelessly associated to the emitter (7), provided with fixing/hanging means, a receiver device (8) provided with an internal memory for storing the data captured by the circuit (5), as well as at least one connection port (9) to a computer (10) for dumping and processing the data by means of a computer application which allows establishing a personal training program for the user, a two-way communication channel being established between the mentioned electronic devices.

## Description

### Object of the Invention

The present invention relates to a shoe which has been specifically conceived to allow its user to have control over different variables presented when walking, such as the distance traveled, the time used, the calories consumed, or the amount or quality of the exercise performed.

The object of the invention is to provide a programmable device with reduced electric consumption, small size and weight, which can be easily removed from the shoe to which it is coupled, allowing the data related to the user to be stored in order to be able to subsequently dump the data in a computer or the like, which through a certain software allows providing a personal training program for the user, advising him or her on guidelines based on the selected objective, such as losing weight, maintaining physical fitness, post-heart attack recovery, reducing triglycerides, etc.

### Background of the Invention

As is known the habit of walking is an exercise from which a number of benefits can be derived if it is practiced in a continuous and controlled manner. Among other benefits which are derived from this practice the following are emphasized:
- Preventing cardiovascular diseases from occurring.
- Recovering after a heart attack.
- Increasing HDL cholesterol (protector). Decreasing triglycerides.
- Decreasing the blood pressure at rest.
- Decreasing and controlling excess weight.
- Increasing bone density (preventing osteoporosis).
- Maintaining physical fitness by means of increasing aerobic capacity.

However the great problem of practicing any physical exercise is abandoning said activity due to the inability of making it a habit, with the necessary continuity and persistence by the individual.

In this sense the solution is to create a faithful link with the individual, for the purpose of motivating him or her to adopt a healthy habit and not to abandon it.

In the field of shoes, a number of devices related to the concept of an "active shoe" are known such as means for monitoring steps, pressure, load and other variables taking part in the activity of walking, as well as shoes provided with means of lighting, vibration and audio, temperature regulation, soles formed by temperatures, etc, the huge majority of which have not been as well received in the market as expected, their success being rather limited.

The applicant himself is the owner of the invention patent P200700705, in which a shoe which allows motivating its user to exercise continuously and regularly, specifically the habit of walking, allowing him/her to follow the evolution of his/her physical shape over time, both over the short term and long term, is described.

More specifically, said shoe has the particularity that in the sole there is provided a practicable package internally provided with an electronic circuit, in which a step detector is provided according to the time and an emitter, such that the mentioned shoe is complemented with a receiver device wirelessly associated to the emitter, provided with fixing/changing means, a receiver device provided with an internal memory for storing the data captured by the circuit, as well as at least one connection port to a computer for dumping and processing the data by means of a computer application which allows establishing a personal training program for the user.

The fundamental problem of this device is that it is impossible to modify its operating parameters once it is installed, since it has a one-way connection port.

### Description of the Invention

The device for the shoe proposed by the invention fully and satisfactorily solves the drawbacks previously set forth since it allows changing the operating characteristics both of the sensor element and the receiver element, allows varying parameters such as the sensor sampling frequency, the volume and the frequency of both stored and collected data, the emission power, therefore allows regulating power consumption, therefore providing a shoe allowing its user to carry out a continuous follow-up of his or her physical activity related to the act of walking, allowing a follow-up of the evolution of the data obtained when walking to be carried out, resulting in a dynamically adaptive interactive device according to, the profile of the user, which allows establishing goals/hallenges for the user with a personalized training program.

To that end the proposed shoe starts from the basic structure of the aforementioned patent, from a shoe which incorporates a self-contained electronics device in a protective package in its sole , which is perfectly integrated in the aesthetics of said sole in the coupling arrangement, a package in which an electric circuit basically based on an electronic circuit provided with a step detector with regard to time is provided, said circuit being wirelessly associated with a small-sized receiver device, which can be established in the belt of the user, integrated in a bracelet, in a necklace or simply in one of the pockets of the user, which collects the data generated while exercising and stores it for later analysis.

Said receiver device incorporates means for connecting to a computer in order to download the mentioned information, the invention being complemented with personal training software for the user, although it can also incorporate a small LED display showing the number of steps taken by the user or an LED bar as an economical alternative and optionally substituting said display.

The data collected during the use of the shoe are processed by the mentioned programming software in order to calculate a series of parameters which allow informing the user about the physical activity being carried out and advising him or her on guidelines based on the selected objective (losing weight, maintaining physical fitness, post-heart attack recovery, reducing triglycerides, decreasing blood pressure at rest, etc.).

The mentioned application can work both through a web environment as well as without the need of an Internet connection in the computer in which it is installed, all this with an attractive design, dynamic adaptation to the profile of the user and allowing him or her to establish goals/challenges which capture his/her attention and prevent him/her from abandoning the exercise.

According to the essence of the invention, among the connection means among the three electronic elements involved in the invention, step detector, receiver device and PC, there is established a two-way communication channel through which it is possible to reprogram the device at any time deemed appropriate, being able to vary the sampling frequency for example, in order to reduce electric consumption, increasing the autonomy thereof.

A device is thus achieved which allows establishing the habit of walking in a simple and attractive manner, from which a number of benefits can be derived for the health of the user.

### Description of the Drawings

To complement the description being made and for the purpose of aiding to better understand the features of the invention according to a preferred practical embodiment thereof, a single page of drawings is attached as an integral part of said description, in which the different elements taking part in the intelligent shoe of the invention have been schematically depicted with an illustrative and non-limiting character.

### Preferred Embodiment of the Invention

In view of the indicated figure it can be observed how the proposed shoe is formed from an upper (1), and a sole (2), both suitable for any line of design, with the special particularity that the mentioned sole (2) includes a housing (3) in which a preferably practicable package (4) is provided, which package contains an electronic circuit (5), with very low electric consumption, provided with a step detector (6) and an emitter (7), such that said circuit (5) wirelessly sends the information relating to the number of steps according to the time passed to a receiver device (8) responsible for storing said information, and which will be discussed further on.

The mentioned electronic circuit (5) will be supplied by the corresponding battery or batteries, not shown in the drawings, having been provided that the autonomy thereof is not less than 6 months, having a compact architecture, a very small size, being integrally housed within the package (4) which will lack outer electronic elements.

The practicable, self-contained and removable features of said package (4) allows the shoe to be used to pass through common security controls in airport checkpoints, bus stations or train stations and/or protected buildings, such that by means of removing said package from the sole (2) of the shoe, it can be used as a conventional shoe with no problem when passing through said checkpoints. For such purpose it has been provided that the shoe has a second package with similar outer features to the package (4) but without inner electronic devices, in order to allow sealing the housing which is visible upon uncoupling said package (4).

The ability to remove the electronic device in a simple and accessible manner enables clearing security barriers with for electronic device detectors commonly used in airports, train stations and (or bus stations and official buildings.

The step detector (6) can be base on five different technologies without affecting the essential nature of the invention. In this sense it can be formed from infrared sensor and emitter, ultrasonic sensor and emitter, piezoelectric sensor, push-button reed switch or resistive ink sensor technologies, whereas the wireless communication (7) is carried out in a miniature transmission module in any of the frequencies fit for such purpose.

The position of the housing (3) will depend on the type of sensor of those previously described, such that it can be located indistinctly in the heel or waist area.

Returning to the receiver device (8), this will be integrated within a protective casing, corresponding with reference number (8), preferably with parallelepiped geometry, of a small size, low weight, with the possibility of being fixed to the belt, waist of the pants, also being able to be carried in a pocket or having hanging means.

Said receiver device (8) gathers the data generated during exercising and keeps it stored in an internal memory to be downloaded through a connection port (9) to a PC (10), preferably a USB port.

Through the mentioned USB port, there is established a two-way communication channel between the three elements: the PC, receiver device and step detector. Said communication channel allows changing the operating characteristics both of the sensor element and the receiver element, varying basic operating parameters such as the sensor reading frequency, the volume and frequency both of collected and/or stored data, or the emission power; and therefore affecting and varying the amount of energy consumed. In short, this possibility of two-way communication allows updating the firmware of the step detector and/or the receiver device, providing the unit with operative and functional flexibility, adapting the characteristics thereof to the special needs of the user.

The receiver device (8) can optionally have a screen or display (11) through which the number of steps taken by the user is shown, or it can incorporate an LED bar.

The data collected during the use of the shoe and once it is dumped into the computer (10) are processed by means of a computer application by way of a personal trainer, designed to be used in a web environment, either on-line or off-line.

Said application is responsible for calculating a multitude of parameters from the two variables which the device collects, the number of steps per unit of time, such as the distance traveled, calories burned, type of exercise and quality thereof, etc, informing the user about the physical activity carried out and advising him or her on guidelines based on the selected objective from among a series of options (losing weight, maintaining physical fitness, post-heart attack recovery, reducing triglycerides, etc). The ultimate objective of that application is to, by means of analyzing the data recorded in the shoe, provide the user with some activity guidelines leading to an increase in his/her quality of life, adhering to the advice issued by the World Health Organization indicating that performing light physical activity, such as walking for example, for at least 30 minutes a day is enough to improve quality of life.

The application will preferably have an attractive graphical design, with the use of flash technologies in order to provided dynamism to the page, dynamically adapting itself according to the profile of the user such that the application has the appearance of a virtual entity in its interaction with the user, it being in charge of making said user loyal to his or her physical activity routine, creating good feelings based on close and personal communication. Said application must also establish goals/challenges for the user, allowing a follow-up of the evolution of the data to be carried out, storing series over the short term and long term for the purpose of being able to compare the results and making interaction with other users possible.

Said application can be linked to a web maintenance service stored in a conventional server.

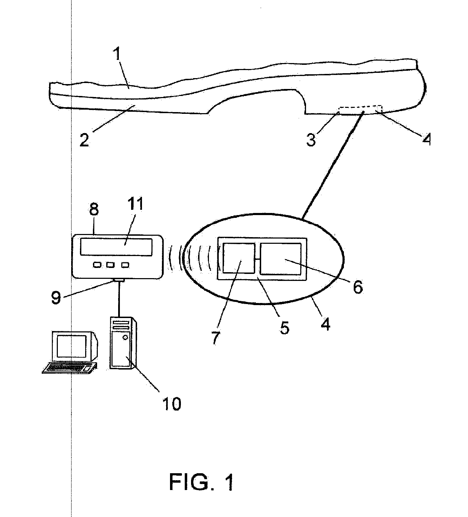

## Claims

1. A programmable intelligent shoe being of the type made of an upper (1) and a sole (2), suitable with any line of design, in which sole there is provided an electronic circuit (5), of low electric consumption, provided with a step detector (6) according to the time and an emitter (7) in the form of a self-contained and removable device, the shoe being complemented with a receiver device (8) wirelessly associated to the emitter (7), provided with an internal memory for storing the data captured by the circuit (5), as well as at least one connection port (9) to a computer (10) for dumping and processing the data by means of a computer application, **characterized in that** the receiver device (8) is provided with a preferably USB type connection port (9) to a PC (10), a two-way communication channel for updating the firmware of the electronic devices, as well as for modifying the characteristics of use thereof such as the sensor reading frequency, the amount and quality of data stored, the power of the emitted signal and similar parameters being defined.

2. The intelligent shoe according to claim 1, **characterized in that** the step detectors (6) are indistinctly based on infrared sensor and emitter, ultrasonic sensor and emitter, piezoelectric sensor, push-button reed switch or resistive ink sensor technologies.

3. The intelligent shoe according to claim 1, **characterized in that** the means for wireless communication between circuits (5-8) are carried out in a miniature transmission module in frequencies fit for such purpose according to the laws in force on wireless transmissions in the place of use.

4. The intelligent shoe according to the previous claims, **characterized in that** the receiver device (8) optionally has a screen or display (11) for displaying the information collected, or in its absence an LED bar.

5. The intelligent shoe according to claim 1, **characterized in that** the receiver device (8) is integrated in a small-sized casing provided with fixing/stabilizing and/or hanging means.
